# EUROPEAN PATENT APPLICATION

(11) **EP 4 830 704 A1**
(43) Date of publication of application: **28.10.2026**
(21) Application number: 24908084.7
(22) Date of filing: 18.12.2024
(51) Int. Cl.: A61K 9/16, A61K 38/13, A61K 9/12, A61K 9/00, A61P 37/06

(54) **METHOD FOR PREPARING BIOADHESIVE PARTICLES FOR IMMUNOSUPPRESSION, PARTICLES PREPARED USING SAME, AND LOCAL IMMUNOSUPPRESSION COMPOSITION THAT IS ADHESIVE TO SURFACES OF TRANSPLANTED ORGAN TISSUE, COMPRISING PARTICLES**

(30) Priority: 18.12.2023 KR 20230184101
(71) Applicant: POSCO Holdings Inc., Pohang-si, Gyeongsangbuk-do 37859 (KR); POSTECH Research and Business Development Foundation, Pohang-si, Gyeongsangbuk-do 37673 (KR)
(72) Inventor: CHA, Hyung-Joon, Pohang-si, Gyeongsangbuk-do 37673 (KR); LEE, Sang-Min, Pohang-si, Gyeongsangbuk-do 37673 (KR); WOO, Hyun-Tack, Pohang-si, Gyeongsangbuk-do 37673 (KR); LEE, Hyeok-Jun, Pohang-si, Gyeongsangbuk-do 37673 (KR); CHOI, Geun-Ho, Pohang-si, Gyeongsangbuk-do 37673 (KR)
(74) Representative: Pfenning, Meinig & Partner mbB
(86) International application number: PCT/KR2024/020642
(87) International publication number: WO 2025/135801

(57) **Abstract**

The present invention relates to a method for preparing bioadhesive particles for immunosuppression, particles prepared using same, and a local immunosuppression composition comprising same. More specifically, the present invention relates to a method for preparing bioadhesive particles for immunosuppression, particles prepared using same, and a local immunosuppression composition comprising same, the method comprising the steps of: preparing a first solution including a biocompatible oil and a surfactant; preparing a second solution, which is a precursor solution including mussel adhesive protein, an immunosuppressive agent, a photocrosslinkable compound and a solvent; and photocrosslinking a mixture of the first solution and the second solution.

## Description

### Technical Field

The present disclosure relates to a method for preparing bioadhesive particles for immunosuppression, particles prepared using same and local immunosuppression compositions including the same. More specifically, the present disclosure relates to a composition for preparing bioadhesive particles containing an immunosuppressive agent based on a bioadhesive material such as mussel adhesive protein and for locally delivering the particles to a transplanted organ.

### Background Art

Immunosuppressive agents are pharmaceutical compounds that depress the activity of the immune system. Immunosuppressive agents are generally used in the treatment of autoimmune diseases and in prophylaxis and therapy related to organ transplantation. After an organ transplant, the recipient's immune system almost invariably recognizes the new organ as foreign and potentially hostile due to a mismatch in human leukocyte antigens between donor and recipient, and attempts to eliminate it by attacking and destroying its cellular components.

More particularly, the organ transplantation technology described above refers to a technique of transplanting an allogeneic, xenogeneic or artificially formed organ for effective regeneration when it is inevitable to replace damaged organs and tissues that are difficult to regenerate due to accidents and aging. However, during the transplantation process, the immune system recognizes the transplanted organ as a foreign object and triggers an immune response that can lead to graft rejection, causing inflammation and damage to the transplanted organ or even complete destruction. Therefore, additional treatment methods are needed to prevent immune responses at the site after organ transplantation surgery.

Currently, the immune response is controlled clinically by administration of immunosuppressive agents. Some examples of immunosuppressive agents include calcineurin inhibitors such as cyclosporin and tacrolimus; corticosteroids; antimetabolites such as azathioprine and mycophenolate; and mTOR inhibitors such as sirolimus. These substances are often not effective alone for treatment, so combination therapies are used to achieve effective suppression. However, these drugs act systemically rather than locally at the site of transplantation, making transplant recipients more susceptible to infection and other diseases, and can cause serious side effects in patients.

In addition, for example, macrolide immunosuppressive agents such as tacrolimus, sirolimus and everolimus are highly active when absorbed into an organism or target tissue, but it is difficult to formulate the compounds or deliver them to a site of action, particularly due to their poor solubility and relatively large molecular size. Meanwhile, for systemic therapy via oral or intravenous routes of administration, immunosuppressive agents are typically presented in soluble formulations including significant amounts of solubilizing excipients such as surfactants and organic solvents.

Accordingly, it is expected that if a technique capable of achieving local immunosuppression in the vicinity of an implanted organ can be provided, this will have wide applicability in the relevant field.

### Disclosure of Invention

### Technical Problem

An aspect of the present disclosure is to provide a method for preparing bioadhesive particles for immunosuppression that can be used in local compositions.

Another aspect of the present disclosure provides an immunosuppressive particle prepared using the method described above.

Another aspect of the present disclosure is to provide a local immunosuppression composition including bioadhesive particles for immunosuppression.

### Solution To Problem

According to an aspect of the present disclosure, a method for preparing immunosuppressive bioadhesive particles includes: preparing a first solution including biocompatible oil and a surfactant; providing a second solution which is a precursor solution including mussel adhesive protein, an immunosuppressive agent, a photocrosslinkable compound, and a solvent; and photocrosslinking a mixture of the first solution and the second solution.

According to another aspect of the present disclosure, an immunosuppressive adhesive particle obtained by the present disclosure is provided.

According to another aspect of the present disclosure, there is provided an adhesive local immunosuppression composition including the immunosuppressive adhesive particles.

### Advantageous Effects Of Invention

According to an aspect of the present disclosure, it is possible to implement local but long-term immunosuppression and thereby stably suppress immune rejection in a process of xenotransplantation. The local immunosuppressive technology according to the present disclosure can more effectively prevent graft rejection without systemic side effects, thus increasing success rates in organ transplantation. In addition, the present disclosure reduces surgical preparation time and additional treatment procedures by using an immunosuppressive agent delivery formulation, thereby reducing medical time and cost. In particular, development of a new immunosuppressive drug delivery formulation with reduced side effects and prolonged duration through local immunosuppression may have significant effect on the field of organ transplantation.

### Brief Description Of Drawings

FIG. 1 is a photograph of the adhesive microgel particles prepared in Example 1 of the present disclosure, observed with a scanning electron microscope (a) and a confocal microscope (b), and particle diameter distribution (c).
FIG. 2 is the results of analyzing the adhesion ability of microgel particles prepared according to Example 1 using a quartz crystal microbalance (QCM).
FIG. 3 is photographs of microgel particles prepared according to Example 1 before (a) and after (b) compression spraying through a spray nozzle as observed by a confocal microscope, and the change in particle diameter (c).
FIG. 4 is photographs of the spray prepared according to Preparation Example 1 before being sprayed onto pig skin tissue and after 7 days and 14 days.
FIG. 5 is a photograph showing the spray prepared according to Preparation Example 1 locally sprayed on pig skin tissue, observed using an optical camera (left) and an optical microscope (right).
FIG. 6 is the loading efficiency of an immunosuppressive drug, cyclosporin A, into microgel particles prepared according to Example 1, analyzed using a UV absorbance spectrophotometer.
FIG. 7 is results of analyzing the release efficiency of the immunosuppressive drug cyclosporin A from microgel particles prepared according to Example 1 using a UV absorbance analyzer including result (a) measured released drug concentration using the UV absorbance analyzer and result (b) analyzed the predicted time for complete drug release using the Higuchi model (b).
FIG. 8(a) is a schematic diagram showing the spray application process in skin xenograft, and FIGS. 8(b) and 8(c) illustrate the result measured by an optical camera and a fluorescence in vivo imaging system (FOBI), respectively.
FIG. 9 is the results of ELISAs analysis of blood collected from a mouse xenograft model, showing the (a) IL-2 concentration and (b) IFN-γ concentration at 1 week and 3 weeks after transplantation.
FIG. 10 is the results of analyzing the spleen of a mouse xenograft model by flow cytometry.
FIG. 11 is the results of analyzing skin tissue harvested from a mouse xenograft model, including photographs (a) taken with an optical microscope after hematoxylin and eosin staining and (b) measurement of area ratio of inflammation.

### Best Mode for the Invention

Hereinafter, a preferred embodiment of the present disclosure will be described with reference to the accompanying drawings. However, it should be noted that the embodiments of the present disclosure may be modified in various other forms and are not limited to the following description.

The present disclosure relates to a spray-based long-term surface coating formulation technology including particles containing bioadhesive proteins. The particles containing the bioadhesive protein of the present disclosure may be sprayable adhesive micro-sized gel particles loaded with immunosuppressive drugs.

The method for preparing the bioadhesive particles of the present disclosure includes operations of: preparing a first solution including biocompatible oil and a surfactant; providing a second solution which is a precursor solution including mussel adhesive protein, an immunosuppressive agent, a photocrosslinkable compound, and a solvent; and photocrosslinking a mixture of the first solution and the second solution.

In each of the above operations, stirring may preferably be performed, for example, stirring may be performed at a rate of 500 to 1000 rpm. More preferably, the mixture of the first solution and the second solution is stirred at 600 to 800 rpm to form micro-sized gel particles.

The biocompatible oil of the present disclosure may be at least one oil selected from the group consisting of mineral oil, oleic acid and Fluorinert FC 40 oil, for example, mineral oil.

Meanwhile, the surfactant used in the present disclosure may be at least one nonionic surfactant selected from the group consisting of sorbitan monolaurate (Arlacel 20), sorbitan monopalmitate (Span-40), sorbitan monooleate (Span-80), sorbitan monostearate, and sorbitan tristearate, and for example, sorbitan monooleate (Span-80) may be used.

The first solution may include the surfactant in an amount of 1 to 10 wt%, preferably the surfactant in an amount of 3 to 5 wt% based on a total weight of the first solution. When the content of the surfactant is less than the above range, there is a problem that a stable emulsion cannot be formed, and when the content exceeds the above range, it causes problems such as viscosity change of the solution and bio-toxicity.

The mussel adhesive proteins that can be used in the present disclosure may be proteins derived from a family of mussels. Preferably, the mussel adhesive proteins include, but are not limited to, those derived from Mytilus edulis, Mytilus galloprovincialis or Mytilus coruscus, or variants thereof. For example, the mussel adhesive proteins may be one or more selected from the group consisting of Mefp (Mytilus edulis foot protein)-1, Mgfp (Mytilus galloprovincialis foot protein)-1, Mcfp (Mytilus coruscus foot protein)-1, Mefp-2, Mefp-3, Mgfp-3 and Mgfp-5, which are derived from mussels. In addition, the mussel adhesive proteins of the present disclosure may be any one selected from all the mussel adhesive proteins described in WO2006/107183 A1 and WO2005/092920 A1, but not limited thereto.

Meanwhile, the immunosuppressive agent that may be included in the second solution may be at least one selected from the group consisting of a purine synthesis inhibitor such as azathioprine, a folic acid antagonist such as methotrexate, macrolides, cyclosporin, tacrolimus, pimecrolimus, everolimus, sirolimus, deforolimus, everolimus, temsirolimus, zotarolimus, infliximab, etanercept, rituximab, tocilizumab, and abatacept, but is not limited thereto.

Meanwhile, the photocrosslinkable compound may include a photo-reactive metal ligand and an electron acceptor.

In the present disclosure, the photo-reactive metal ligand for providing a molecule strongly absorbing visible light may be one or more selected from the group consisting of ruthenium (Ru(II)), palladium (Pd(II)), copper (Cu(II)), nickel (Ni(II)), manganese (Mn(II)), and iron (Fe(III)). For example, it is preferable to use [Ru(II)bpy₃]Cl₂, but not limited thereto.

In addition, the photocrosslinkable compound may further include one or more selected from the group consisting of sodium persulfate, periodate, perbromate, perchlorate, vitamin (B12), pentaamminechlorocobalt (III), ammonium cerium (IV) nitrate, oxalic acid and EDTA to provide an electron acceptor. For example, it is preferable that the photocrosslinkable compound uses sodium persulfate, but not limited thereto.

More preferably, the photocrosslinkable compound may be a combination of [Ru(bpy)₃]²⁺ and sodium persulfate, in which case blue light such as light having a wavelength of 400 to 500 nm or 420 to 480 nm is irradiated to form a bioadhesive material having adhesiveness in the form of gel particles.

More specifically, for preparing the immunosuppressive bioadhesive particles of the present disclosure, photocrosslinking is preferably performed while mixing the first solution and the second solution, for example, by stirring.

In this case, the mixture of the first solution and the second solution may be obtained by mixing the first solution and the second solution at a volume ratio of 5 to 15:1, for example, at a volume ratio of 8 to 12:1, when the first solution is contained in an amount less than the above range, there is a problem in that the droplets are not well separated during stirring, making it difficult to control the particle size, and, when the amount exceeds the above range, the precursor solution is not well separated during stirring, thereby limiting particle formation.

Meanwhile, the solvent that can be used in the method for preparing the bioadhesive particles of the present disclosure may be at least one selected from the group consisting of a physiological saline solution such as PBS, an organic solvent and a contrast agent, and may be used alone or in combination with two or more thereof. For example, the solvent of the present disclosure may be an aqueous solution containing 20 to 40 v/v % of DMSO.

The second solution may include, based on the total weight of the second solution, 2 to 10 wt% of mussel adhesive protein and 0.01 to 5 wt% of an immunosuppressive agent, and a balance being a solvent. When the content of the mussel adhesive protein is less than the above range, adhesion of particles may be insufficient, and when it exceeds the above range, particle size may increase or particle shape may not be smoothly obtained. Meanwhile, when the content of the immunosuppressive agent is less than the above range, immunosuppressive effect may be insufficient, and when it exceeds the above range, particle shape may not be smoothly obtained.

The second solution may include the photo-reactive metal ligand in an amount of 0.1 to 5 mM, more specifically, 1 to 3 mM, and when the concentration of the photo-reactive metal ligand is within this range, it provides a sufficient reaction rate for photocrosslinking and enables subsequent initiator removal process to be performed stably and effectively.

In addition, the second solution may include 1 to 20 mM of the electron acceptor, for example, 5 to 15 mM, and when the concentration of the electron acceptor is in the above range, micro-sized fine particles can be stably formed.

According to another aspect of the present disclosure, an immunosuppressive adhesive particle that is obtained by the method for preparing bioadhesive particles for immunosuppression is provided.

The particle size obtained by the present disclosure may be in a range of 1 to 200 µm and have an average diameter of 3 to 15 µm, for example, 5 to 12 µm. When particles having such a size are formed, stable spraying is possible, so that they can be applied as a spray formulation.

According to another aspect of the present disclosure, there is provided a local immunosuppression composition including the immunosuppressive adhesive particles according to the present disclosure.

When the local immunosuppression composition of the present disclosure is used, the immunosuppressive drug loaded in microparticles can be continuously released while maintaining particles to shorten a dosage interval.

In this case, the local immunosuppression composition may include, based on the total volume of the composition, 60 to 90 v/v % of the immunosuppressive adhesive particles and a balance being a solvent, for example, 65 to 85 v/v % of the immunosuppressive adhesive particles and a balance being a solvent may be included. When the amount of the immunosuppressive adhesive particles is less than the above range, a large amount of the composition should be applied in order to coat or apply the entire surface when applied to an object, and when it exceeds the above range, smooth spraying may not be performed.

The local immunosuppression composition of the present disclosure may be a formulation for spray. More specifically, the local immunosuppression composition of the present disclosure may be formulated into a surface coating spray, wherein the solvent to be used is for example an aqueous ethanol solution, and preferably a mixture of physiological saline solution and ethanol in a volume ratio of 1:5 to 5:9.

The spray may be a compressed spray and thus surface coating can be induced quickly and uniformly through the compression spraying process. The drug delivery based on the composition prepared by formulating the local immunosuppression composition of the present disclosure as a spray formulation has high adhesion strength and retention force of the spray particles themselves, thereby maintaining a stable immunosuppressive effect for a long period of time.

That is, the organ to which the local immunosuppression composition of the present disclosure is applied can be locally surface-coated with the local immunosuppression composition of the present disclosure, and thus the loaded drug may be continuously released in a target organ, thereby enabling long-term action of the drug during an organ transplantation process.

Hereinafter, the present disclosure will be described in more detail with reference to specific examples. The following examples are merely illustrative of the present disclosure and do not limit the scope thereof.

### Mode for the Invention

### Example

### Example 1 : Preparation of adhesive microgel particles loaded with immunosuppressive drugs

For a batch reactor, a 50 ml glass Erlenmeyer flask and magnetic stirring bar were used.

The first solution was prepared by mixing mineral oil (Sigma Aldrich) with a lipophilic surfactant, Span 80 at 3 wt%, based on the total weight of the first solution, followed by stirring in a glass Erlenmeyer flask at rpm 700.

A second solution (interchangeably referred to as a 'precursor solution') was prepared in which 4 wt% (120 mg) of mussel adhesive protein and 0.2 wt% (6 mg) of the immunosuppressive drug cyclosporin A (CsA), each based on the total weight of the second solution, were dissolved together with polymerization initiators [Ru(bpy)3]²⁺ (1 mM) and SPS (10 mM) in 3 ml of a mixed solvent consisting of 2 ml of distilled water (DW) and 1 ml of dimethyl sulfoxide (DMSO).

The first solution and the second solution were added in a volume ratio of 10:1, followed by stirring at 700 rpm for 10 minutes.

Then, the mixture was stirred at 700 rpm for 1 minute while being exposed to blue light to induce photocrosslinking and thus obtain microgel particles.

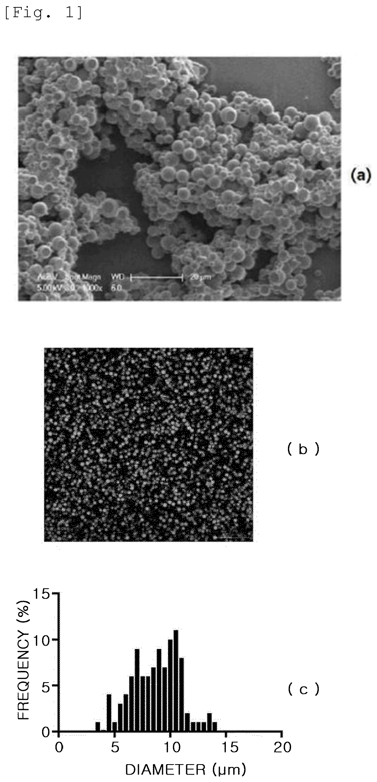

The microgel particles were observed by a scanning electron microscope (a) and a confocal microscope (b), and the results of measuring particle diameters (c) are shown in FIG. 1.

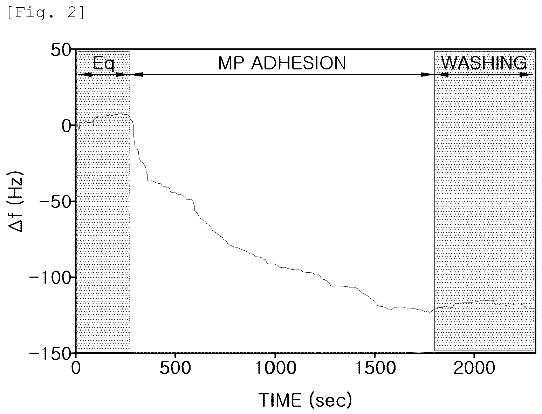

Meanwhile, the adhesion of the microgel particles obtained as described above was measured using a quartz crystal microbalance (QCM), and the results are shown in FIG. 2. As can be seen from FIG. 2, it was confirmed that the particles were attached to the QCM sensor and remained well-adhered even after washing.

### Preparation Example 1 : Preparation of a spray composition based on adhesive microgel particles

The adhesive microgel particles prepared in Example 1 were concentrated to a concentration of 75 v/v % with respect to the volume of the microgel in 2 ml of a solution obtained by mixing PBS and ethanol at a ratio of 3:7 (v/v), thereby preparing a spray composition.

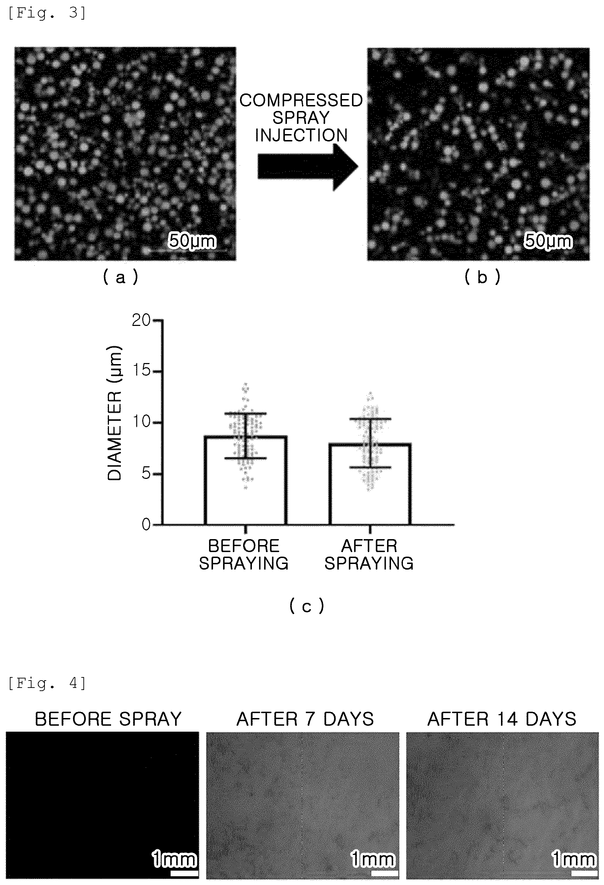

The prepared spray composition was compressed and sprayed through a compressed spray container, and the particles before (a) and after spraying (b) were observed by a confocal microscope, and the change in diameter (c) is shown in FIG. 3.

Referring to the results of FIG. 3, it was confirmed that even after compression injection, the microgel particles of the present disclosure were stably maintained without a change in diameter.

In addition, to confirm the retention force of the spray particles, a spray composition was sprayed onto pig skin tissue having dimensions of 4 cm wide and 2 cm long, dried for 2 minutes, and then stirred in 10 ml of PBS for 2 weeks, and the result was measured using a fluorescence microscope, with the results shown in FIG. 4. As a result, it was confirmed that the surface coating state of pig skin was stably maintained in a physiological aqueous environment for 2 weeks.

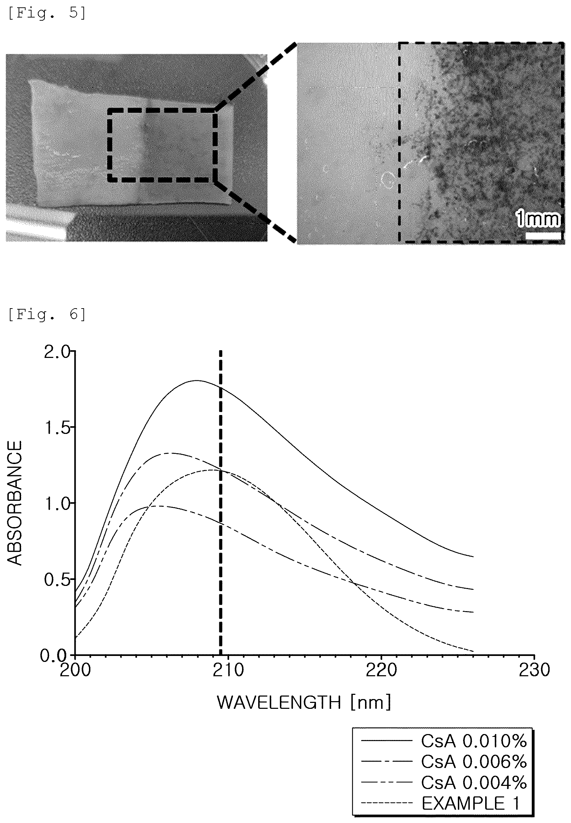

In addition, the microgel particles were locally sprayed on pig skin tissue and coated at desired sites, which was measured by an optical camera (left photo of FIG. 5) and an optical microscope (right photo of FIG. 5), as shown in FIG. 5.

### Experimental Examples

### 1. Immunosuppressive drug loading efficiency and release of adhesive microgel particles

### (1) Immunosuppressive drug loading efficiency

The adhesive microgel particles prepared in Example 1 were lyophilized for one day, then the gel particles were crushed with a mortar and pestle, mixed with distilled water (DW), and absorbance was measured using a UV spectrophotometer, as shown in FIG. 6.

As the results were compared with the absorbance of aqueous solutions of cyclosporin A at concentrations of 0.010%, 0.006%, and 0.004%, the immunosuppressive drug loading efficiency of cyclosporin A in the adhesive microgel particles was confirmed to be 62.4%, calculated as (cyclosporin A inside the microgel / cyclosporin A included in the initial precursor solution) × 100.

### (2) Confirming drug release efficiency

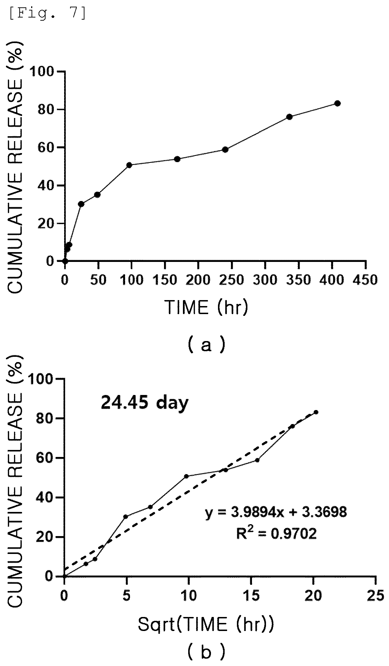

The microgel particles prepared in Example 1 were mixed with 10 ml of PBS and then placed into a dialysis pack having a molecular weight cut-off (MWCO) of 3.5 kD, which was put into a conical tube containing 30 ml of PBS and incubated at 37° C while being stirred. The supernatant was extracted by 1 ml during the incubation for three weeks, and the results (a) of measuring the release drug concentration through a UV absorbance analyzer and (b) of analyzing the expected date of full release through the Higuchi model ( $Q = k \times {t}^{\frac{1}{2}}$) are shown in FIG. 7. As a result, it was analyzed that the expected period for completely releasing the drug loaded on the adhesive microgel particles is about 24 days.

### 2. Animal modeling and spray application of xenograft to observe immune response

The dorsal skin of a rat (Sprague Dawley strain) aged between 6 and 8 weeks was collected after euthanasia, and the size of each sample was about 1 cm × 1 cm. The collected tissue was washed twice with 5 ml of a minimum essential medium (DMEM) containing fetal bovine serum (FBS) and antibiotics, followed by storage in cold physiological saline solution (PBS).

The back skin tissue of anesthetized BALB/c mice aged six weeks or more is cut into a size of 1 cm × 1 cm.

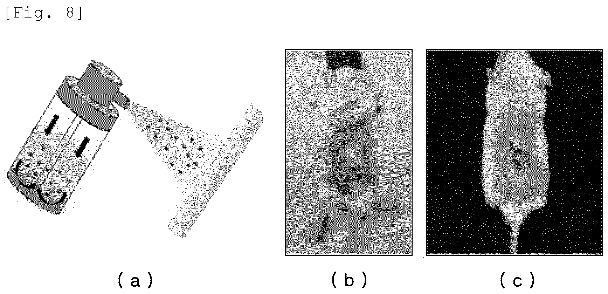

The adhesive microgel spray prepared in Preparation Example 1 was injected into the rat skin tissue at a volume of 1 ml and dried for 2 minutes, then sewn to the incision site of mouse using suture thread as shown schematically in FIG. 8(a). Meanwhile, the results obtained by measuring with an optical camera (b) and fluorescence imaging system (FOBI) (c) are shown in FIG. 8. As a result, it was confirmed that the rat tissue was successfully sewn and the sprayed adhesive microgel particles were maintained at the implantation site.

### 3. Tissue collection and analysis of immune factors for observation of immune response

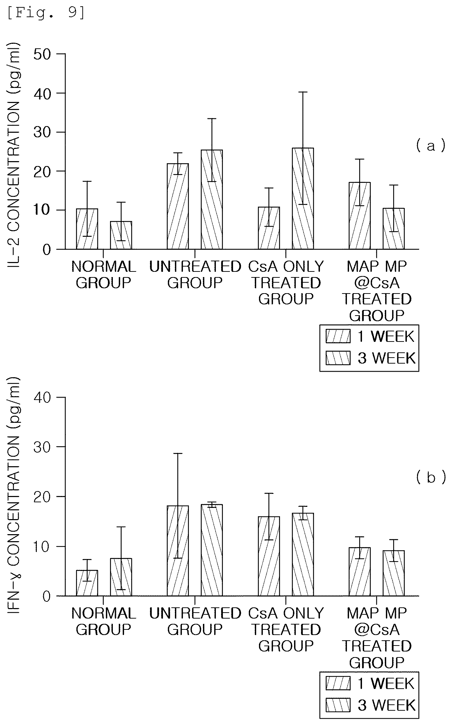

The skin under the rib of the mouse xenograft model prepared in 2. above was cut to a length of 4 cm and then the diaphragm and intercostal space were cut. 0.3 ml to 0.5 ml of blood was collected from the left atrium using a 1 ml syringe. The collected blood was analyzed by ELISA as shown in FIG. 9. As a result of the analysis, it was confirmed that the tissues without any treatment (untreated group) had higher concentrations of IL-2 and IFN-γ than those of normal mice (normal group) at both week 1 and week 3 after transplantation. In case of the tissues injected with only immunosuppressive drugs (CsA only treated group), the concentration of IL-2 in the blood was inhibited at week 1 but not continuously released so that it showed similar results to the negative control group at week 3, and the concentrations of IL-2 and IFN-γ in the blood were significantly inhibited up to week 3 in case of the tissues applied with adhesive microgel particle spray (MAP MP@CsA treated group).

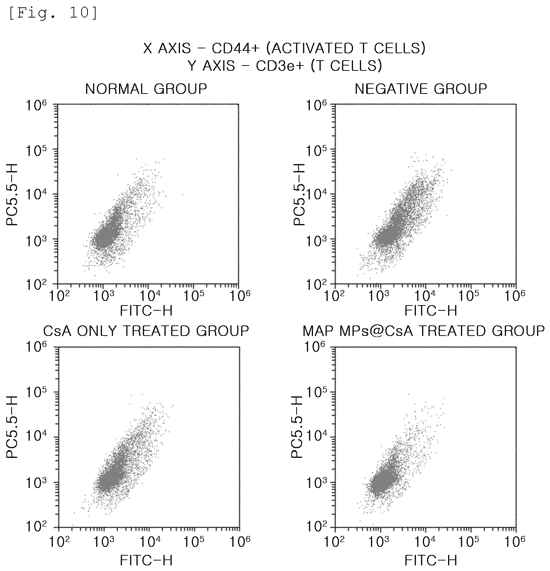

In addition, the epidermis of a point 1 cm from the left hind leg of the mouse xenograft model prepared in 2. above was cut. The spleen was collected by partially cutting the dermis above the spleen and then cutting the connective tissue. The collected spleen tissue was finely minced on a cell strainer, and physiological saline containing cells was obtained by spraying 3 ml of physiological saline solution thereon. The physiological saline solution containing dispersed cells was centrifuged at 2000 rpm for 3 minutes to separate only precipitates, which were then dispersed in flow cytometry buffer (physiological saline solution 90%, fetal bovine serum solution 10%). The flow cytometry solution containing dispersed cells was diluted to have a concentration of 500,000 cell per 1 ml. The diluted flow cytometry solution containing dispersed cells was dissolved with CD3e and CD44 specific fluorescent dyes at 0.001 wt% and stained with the cells in the dark for 30 minutes. The stained cells underwent three successive washing operations of centrifuging the cells at 1500 rpm for 3 minutes and re-dispersing them in 1 ml of flow cytometry buffer. The washed cells were analyzed using a flow cytometer, and the results are shown in FIG. 10 as total T cells (y axis, fluorescence detection result of CD3e) vs T cell activation level (x axis, fluorescence detection result of CD44).

As a result, as shown in FIG. 10, compared to the tissue collected from normal mice (normal group), it was confirmed that the tendency of T cell activation is significantly higher than the total T cell fluorescence detection results in the tissues injected with only immunosuppressive drugs (CsA only treated group) and not treated (negative control group). Meanwhile, it was confirmed that the tissue applied with the adhesive microgel particle spray of Preparation Example 1 loaded with an immunosuppressive drug by the present disclosure (MAP MPs@CsA treated group) showed similar T cell activation tendencies to those of the normal group, and significant immunosuppression effects were observed in terms of cellular activity.

### 4. Collection of transplanted xenogeneic skin tissue and analysis of inflammatory response

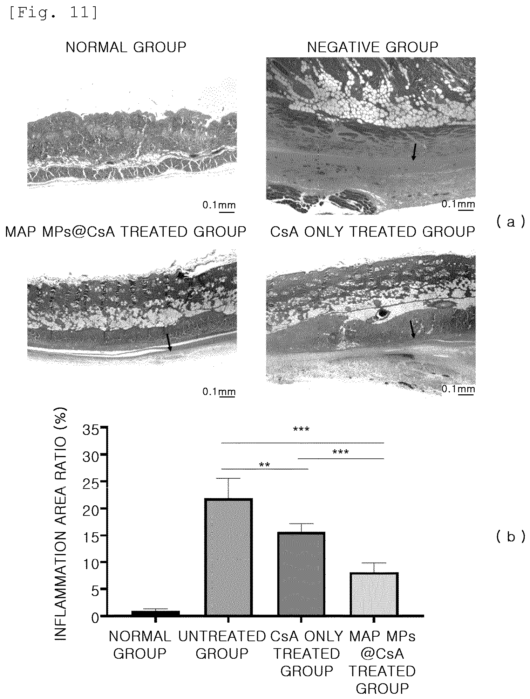

The skin tissue including the host tissue within 0.5 cm around the implantation site of the mouse xenograft model prepared in 2. above was collected, and the tissue of the same location and size was also collected from normal mice aged 6 weeks or older and stored in 10 ml of formalin solution. The collected tissues were stained with H&E, observed under an optical microscope, and analyzed for the area ratio of inflammation using Image J program, and the results are shown in FIG. 11.

As a result, as shown in FIG. 11(a), it was confirmed that the tissues injected with only immunosuppressive drugs (CsA only treated group) and those without any treatments (untreated group) showed severe inflammatory responses compared to the tissues collected from normal mice (normal group). Meanwhile, it was confirmed that the inflammation response was greatly improved in the tissue applied with the adhesive microgel particle spray of Preparation Example 1 loaded with an immunosuppressive drug (MAP MPs@CsA treated group). FIG. 11(b) is a graph showing the results of measuring the area ratio of inflammation.

While examples have been shown and described above, it will be apparent to those skilled in the art that modifications and variations could be made without departing from the scope of the present disclosure as defined by the appended claims.

## Claims

1. A method for preparing bioadhesive particles for immunosuppression comprising the operations of:
preparing a first solution including biocompatible oil and a surfactant;
providing a second solution which is a precursor solution including mussel adhesive protein, an immunosuppressive agent, a photocrosslinkable compound, and a solvent; and
photocrosslinking a mixture of the first solution and the second solution.

2. The method for preparing bioadhesive particles for immunosuppression of claim 1, wherein the biocompatible oil is at least one oil selected from the group consisting of mineral oil, oleic acid and Fluorinert FC 40 oil.

3. The method for preparing bioadhesive particles for immunosuppression of claim 1, wherein the surfactant is at least one nonionic surfactant selected from the group consisting of sorbitan monolaurate (Arlacel 20), sorbitan monopalmitate (Span-40), sorbitan monooleate (Span-80), sorbitan monostearate and sorbitan tristearate.

4. The method for preparing bioadhesive particles for immunosuppression of claim 1, wherein the first solution comprises the surfactant in an amount of 1 to 10 wt% based on a total weight of the first solution.

5. The method for preparing bioadhesive particles for immunosuppression of claim 1, wherein the mussel adhesive protein is at least one selected from the group consisting of Mefp-1, Mgfp-1, Mcfp-1, Mefp-2, Mefp-3, Mgfp-3 and Mgfp-5.

6. The method for preparing bioadhesive particles for immunosuppression of claim 1, wherein the immunosuppressive agent is at least one selected from the group consisting of azathioprine, methotrexate, macrolides, cyclosporin, tacrolimus, pimecrolimus, everolimus, sirolimus, deforolimus, everolimus, temsirolimus, zotarolimus, infliximab, etanercept, rituximab, tocilizumab, and abatacept.

7. The method for preparing bioadhesive particles for immunosuppression of claim 1, wherein the photocrosslinkable compound comprises a photo-reactive metal ligand and an electron acceptor.

8. The method for preparing bioadhesive particles for immunosuppression of claim 1, wherein the solvent is an aqueous solution containing 20 to 40 v/v % of DMSO.

9. The method for preparing bioadhesive particles for immunosuppression of claim 1, wherein the second solution comprises, based on the total weight of the second solution, 2 to 10 wt% of mussel adhesive protein and 0.01 to 5 wt% of an immunosuppressive agent, and a balance being a solvent.

10. The method for preparing bioadhesive particles for immunosuppression of claim 7, wherein the second solution comprises between 0.1 and 5 mM of a photo-reactive metal ligand and between 1 and 20 mM of an electron acceptor.

11. The method for preparing bioadhesive particles for immunosuppression of claim 1, wherein the mixture of the first solution and the second solution is obtained by mixing the first solution and the second solution at a volume ratio of 5 to 15:1.

12. An immunosuppressive adhesive particle obtained by any one of claims 1 to 11.

13. An adhesive local immunosuppression composition comprising the immunosuppressive adhesive particles of claim 12.

14. The adhesive local immunosuppression composition of claim 13, wherein the local immunosuppression composition comprises, based on the total volume of the composition, 60 to 90 v/v % of the immunosuppressive adhesive particles and a balance being a solvent.

15. The adhesive local immunosuppression composition of claim 13, wherein the local immunosuppression composition is a formulation for spray.
